# EUROPEAN PATENT APPLICATION

(11) **EP 2 014 181 A2**
(43) Date of publication of application: **14.01.2009**
(21) Application number: 08156900.6
(22) Date of filing: 26.05.2008
(51) Int. Cl.: A23L 1/308, A61K 31/702, A61K 31/715, A61K 31/733, A61P 1/12

(54) **Compositions based on prebiotic and immunogenic ingredients for the prevention and treatment of gastroenteric disorders caused by dysbiosis and/or alterations of the normal intestinal flora**

(30) Priority: 15.06.2007 IT MI20071214
(71) Applicant: S.I.I.T. S.r.l. Servizio Internazionale Imballaggi Termosaldanti, 20090 Trezzano S/N (Milan) (IT)
(72) Inventor: Di Pierro, Francesco, 20090, TREZZANO SUL NAVIGLIO (MI) (IT)
(74) Representative: Minoja, Fabrizio

(57) **Abstract**

The present invention relates to compositions containing bifidogenic and lactogenic prebiotic ingredients and immunogenic ingredients able to promote the proliferation and colonisation of bifidobacteria and lactic acid bacteria in the intestine, for the prevention and treatment of gastroenteric disorders.

## Description

### FIELD OF INVENTION

The present invention relates to compositions containing prebiotic ingredients and immunogenic ingredients able to promote the proliferation and colonisation of bifidobacteria and lactic acid bacteria in the intestine, for the prevention and treatment of gastroenteric disorders.

### INTRODUCTION

The human intestine, which is sterile at birth, is rapidly colonised by micro-organisms (pyogens, pathogens, fungi and other rarer unicellular organisms) which, in adults, amount to some 10¹⁴ live cells.

They include 10¹⁰ bacteroids (organisms such as B. fragilis and B. subtilis, which are only pathogenic in normal tissues outside the intestine); 10⁹ bifidobacteria; 10⁹ Eubacteriaceae (including coliforms and clostridia which are not necessarily pathogenic); 10⁹ streptococci and 10⁸ lactic acid bacteria.

It is widely recognised by the scientific community that bifidobacteria and lactobacilli favourably influence the intestinal well-being in terms of immunological, digestive, transit, anti-constipation, anti-diarrhoea and nutrient absorption capacity. They can be "fed" with prebiotic fibres, which constitute a kind of nutrient substrate for these bacterial species only. In a healthy intestine, the two strains coexist in the ratio of 10:1.

Fibres such as inulin, galactooligosaccharides (GOS), fructooligosaccharides (FOS), lactosucrose, pyrodextrin, soy oligosaccharides and transgalactooligosaccharides are essentially bifidogenic, whereas fibres like isomaltooligosaccharide, lactilol, lactulose, xylooligosaccharide, mannan oligosaccharide and polydextrose are essentially lactogenic.

### DESCRIPTION OF THE INVENTION

It has now been found that compositions containing:
a) bifidogenic prebiotic ingredients,
b) lactogenic prebiotic ingredients, and
c) immunogenic ingredients,
mixed in suitable proportions, promote species-specific proliferation and colonisation of bifidobacteria and lactic acid bacteria in the intestine.

The present invention therefore relates to compositions for the prevention and treatment of gastroenteric disorders, especially those caused by dysbiosis and/or alterations of the normal intestinal flora due to diarrhoea, antibiotic treatments, dietary imbalances, dyspepsia, malabsorption, autoimmune disorders and colitis.

More particularly, the invention relates to compositions containing:
a) one or more bifidogenic prebiotic ingredients chosen from inulin, fructooligosaccharides (FOS), galactooligosaccharides (GOS), lactosucrose, pyrodextrin, soy oligosaccharides and transgalactooligosaccharides,
b) one or more lactogenic prebiotic ingredients chosen from isomaltooligosaccharide, lactilol, lactulose, xylooligosaccharide, mannan oligosaccharide and polydextrose, and
c) one or more immunogenic ingredients chosen from arabinogalactan and derivatives thereof, plant extracts or extracts of fungi which contain them.

The compositions according to the invention promote the proliferation processes and colonising capacity of bifidobacteria and lactic acid bacteria strains without altering their normal ratios. According to a preferred aspect, the compositions according to the invention will contain the bifidogenic and lactogenic prebiotic ingredients suitably mixed in the ratio of 10:1, in order to reproduce the natural intestinal proportions.

According to a preferred aspect, the compositions of the invention will contain the bifidogenic prebiotic ingredients FOS and GOS in the ratio of 1:9. This ratio reflects their natural proportions in human milk. As GOS and FOS mainly work in the first portion of the colon, and inulin (which is also essentially bifidogenic) mainly works in the lower part thereof, GOS and FOS combined will be present in the ratio of 1:1 to inulin alone. These internal ratios (10:1 bifidogenic to lactogenic bacteria; 9:1 GOS to FOS and 1:1 GOS+FOS to inulin) also take account of the need for fibres which produce an anti-pathogenic effect in the intestine by promoting the strains of bacteria that release butyric acid, as well as acetate, propionate and lactate.

According to a preferred aspect, the compositions of the invention will contain the bifidogenic and lactogenic prebiotic ingredients in the following percentages, taking their global value as 100:
40% GOS
5% FOS
45% inulin
5% isomaltooligosaccharide
2.5% lactulose
2.5% polydextrose.

According to a preferred aspect, the compositions of the invention will contain immunogenic ingredients such as arabinogalactan and derivatives thereof (more generally, simple and branched-chain pectins and derivatives thereof), which may be present in pure form or as plant extracts (such as derivatives of Echinacea, Uncaria or Astragalus) or extracts of fungi containing them. These immunogenic ingredients will be contained in the total composition in the quantity of between 1 and 1000 mg.

According to a particularly preferred aspect, the compositions of the invention will contain the various ingredients in the following percentage intervals:
a) bifidogenic prebiotic ingredients: between 10 and 10,000 mg,
b) lactogenic prebiotic ingredients: between 10 and 10,000 mg, and
c) immunogenic ingredients: between 10 and 10,000 mg.

The compositions according to the invention may also contain other ingredients which promote intestinal well-being even in the event of concomitant disorders. Ingredients of this type are anthraquinone laxatives, osmotic laxatives, mass laxatives (obtained by extraction, semisynthesis or synthesis); mixtures of probiotics (spore-forming and sporeless; inactivated or live); extractive and non-extractive derivatives, with spasmolytic/sedative properties (camomile, valerian, passion-flower, hawthorn, griffonia, tryptophan and derivatives thereof); vitamins and minerals that play a key part in intestinal well-being; beta-glucans and derivatives thereof, yeasts, lysates and derivatives thereof. Each active ingredient could be included in the compositions in a technologically simple form or in the form of phytosomes, liposomes, co-ground products or ter-clatrates. Each active ingredient could also be used in normal-release, time-dependent or pH-dependent forms.

The compositions according to the invention may be formulated suitably for oral administration, and will be prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

The compositions according to the invention could be formulated, for example, in the form of tablets, capsules, granulates, sachets and the like.

Some examples of formulations according to the invention are set out below.

### EXAMPLES

### Example 1

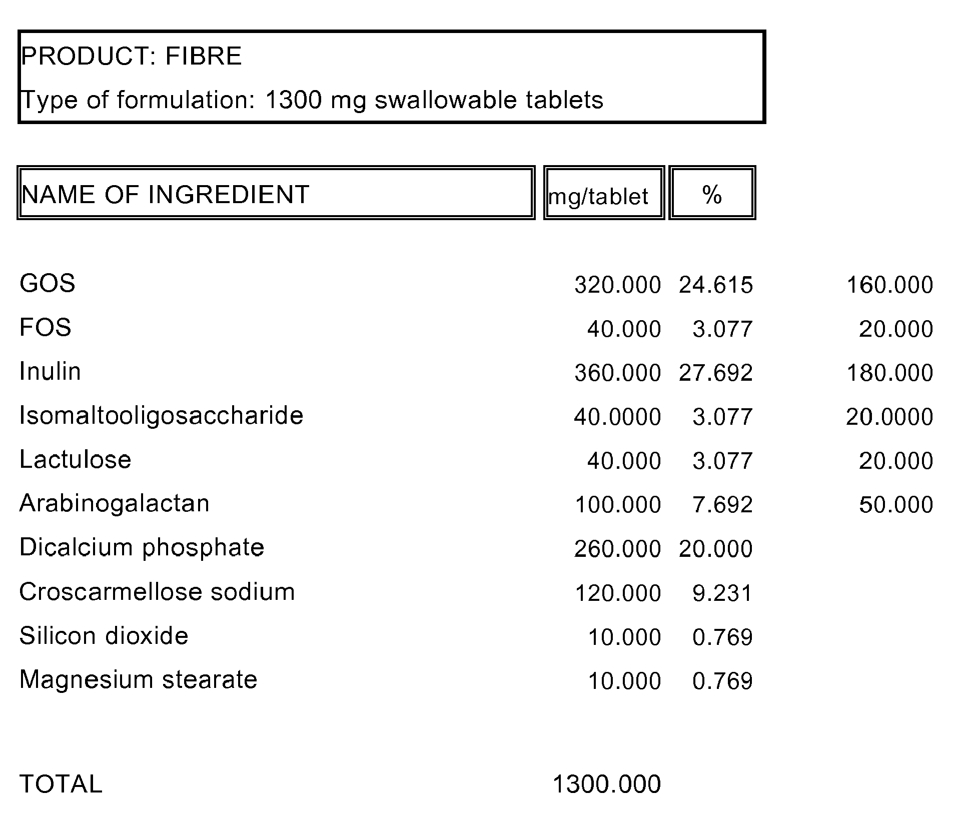

### Example 2

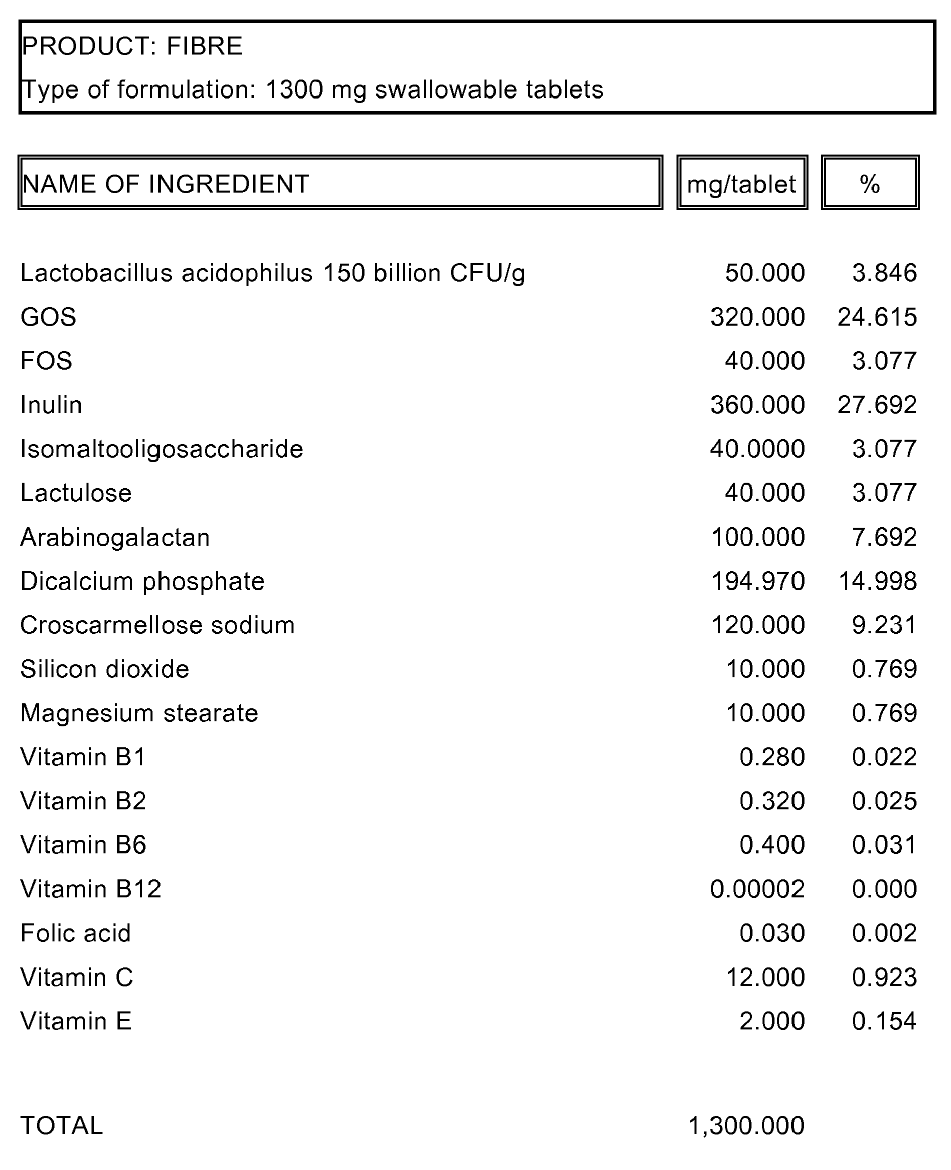

### Example 3

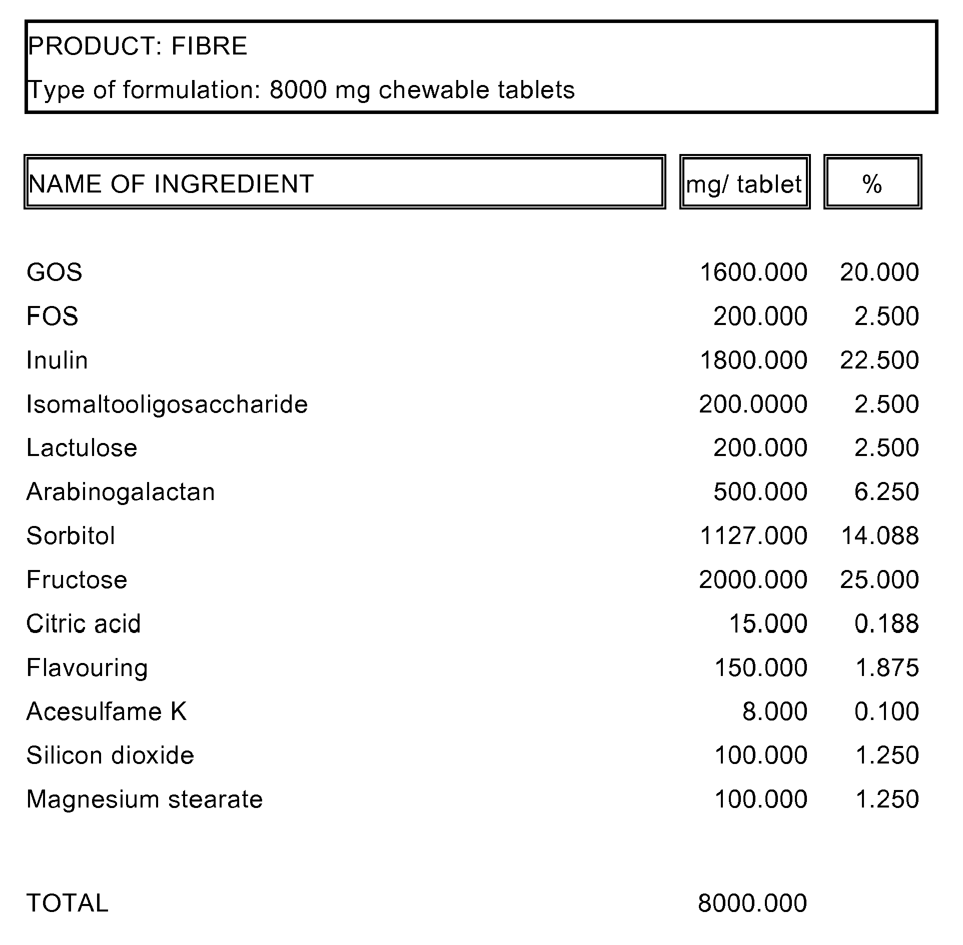

### Example 4

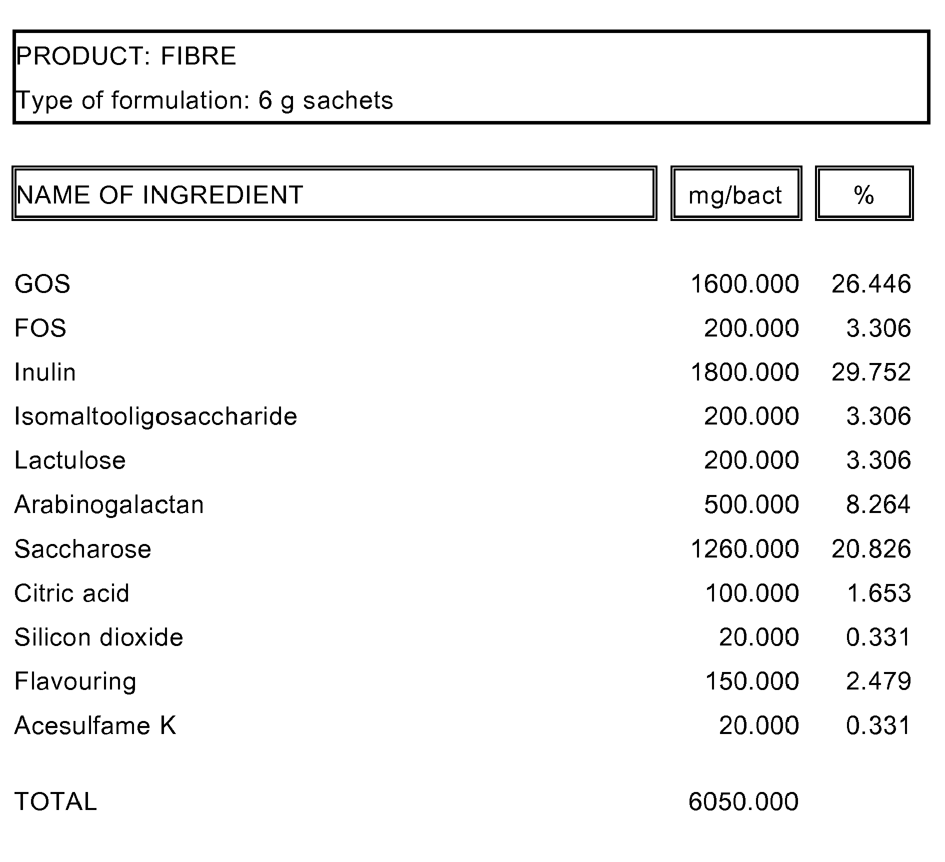

### Example 5

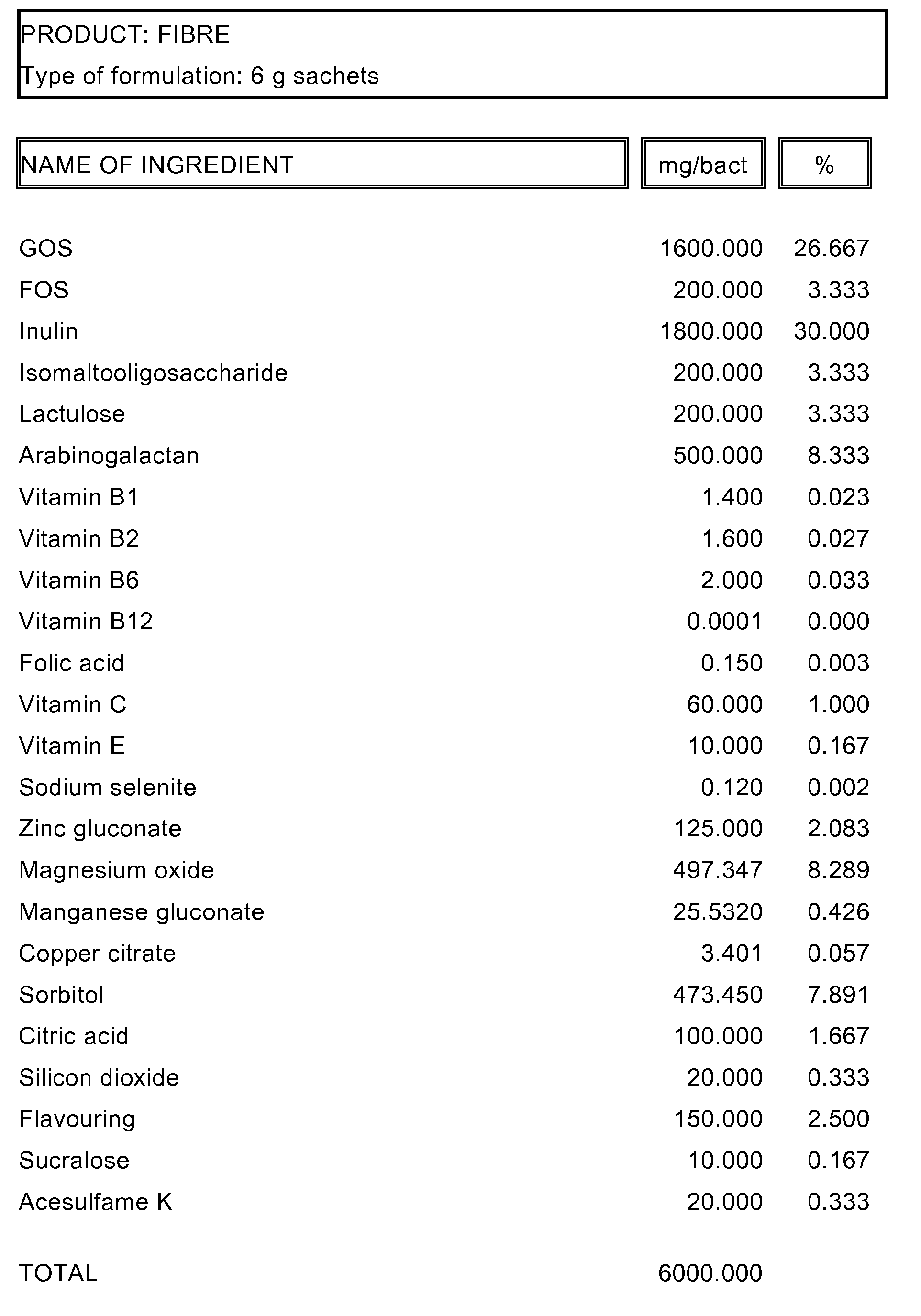

### Example 6

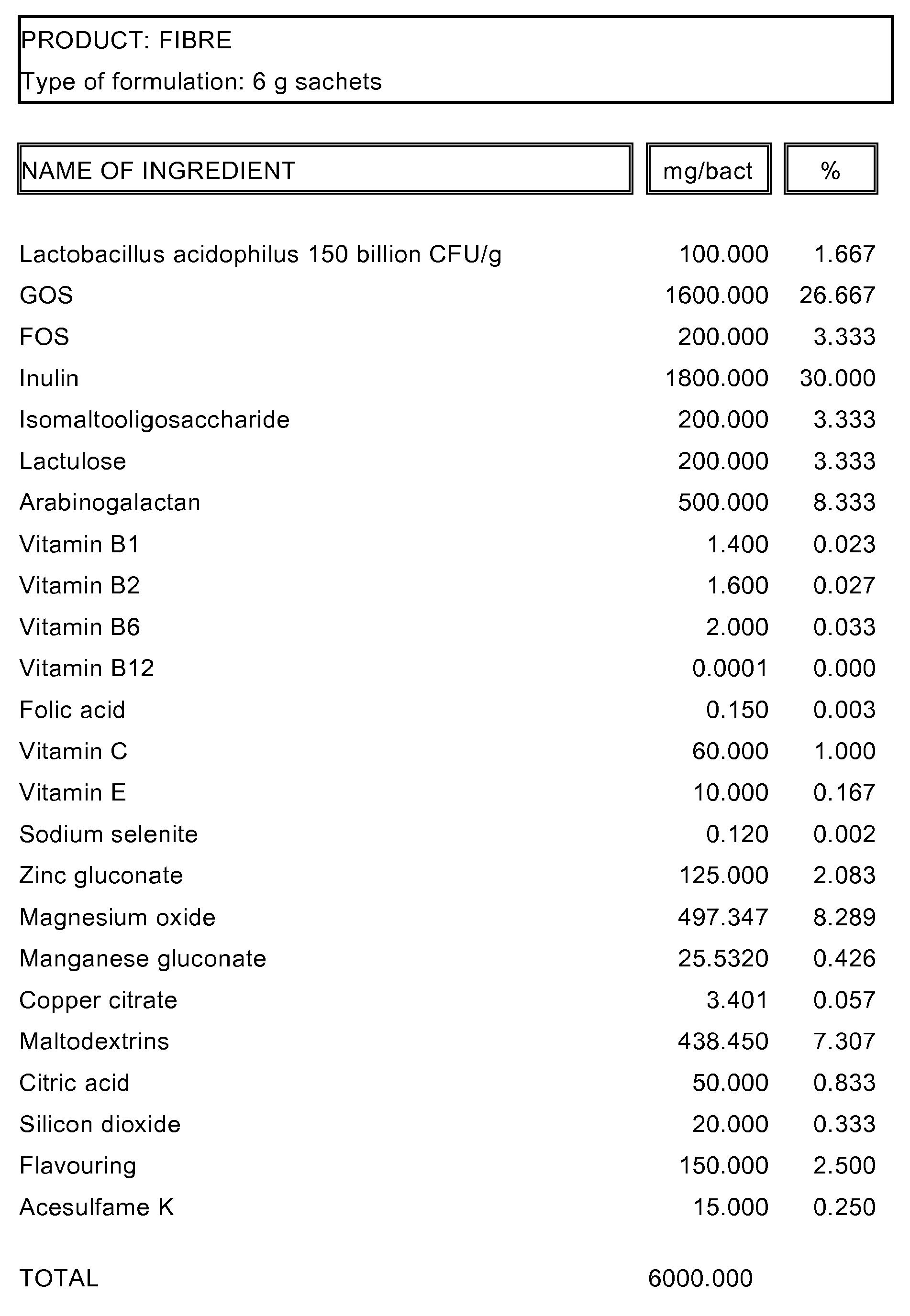

### Example 7

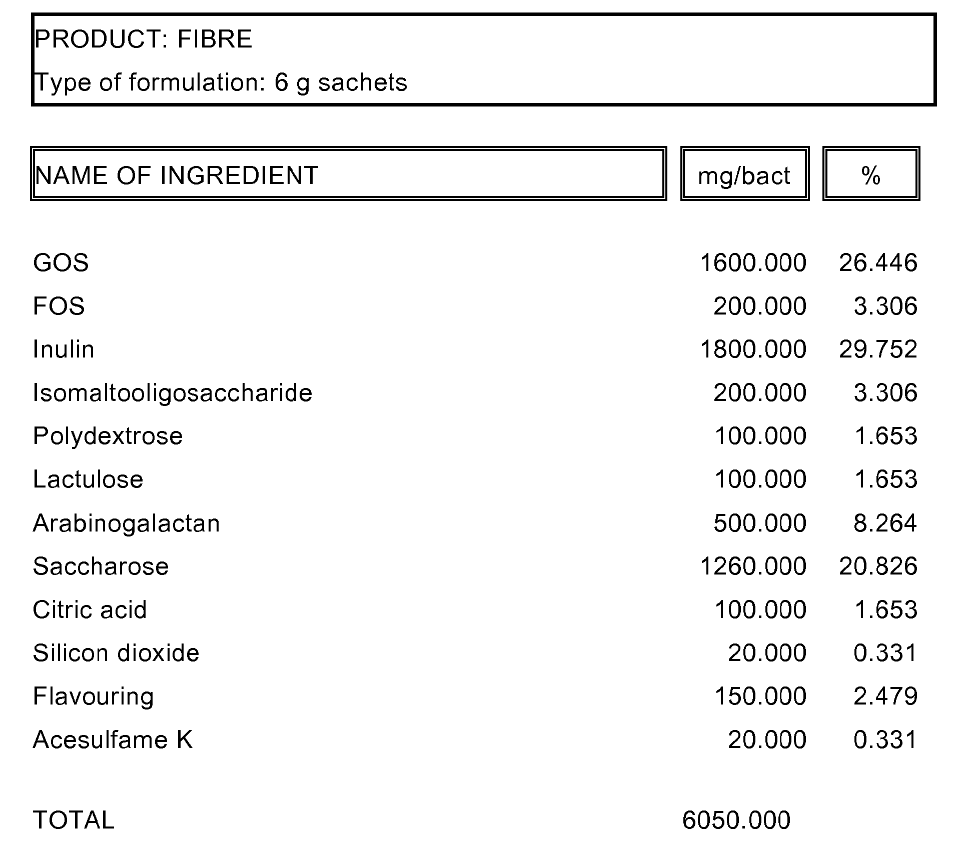

### Example 8

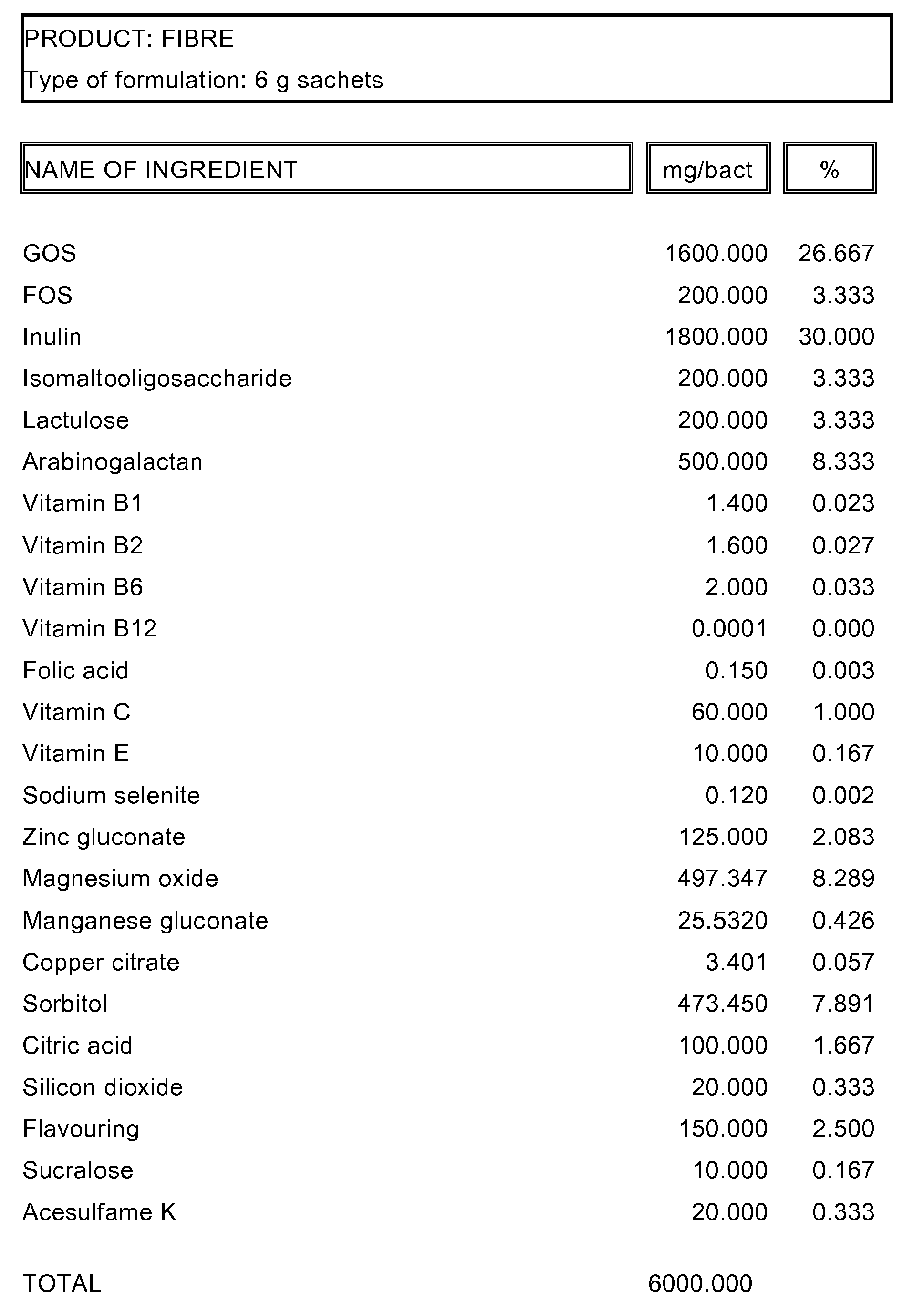

### Example 9

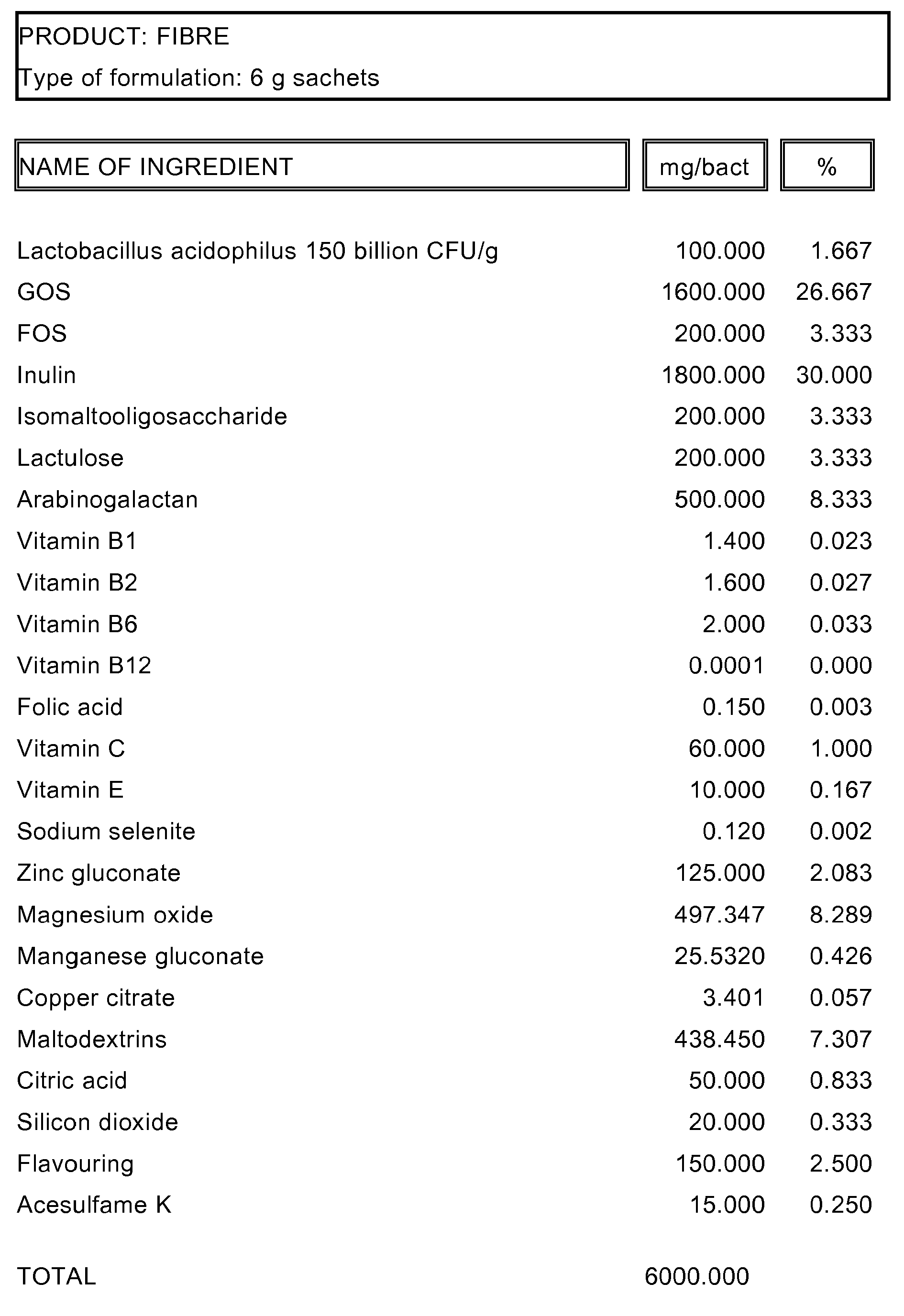

### Example 10

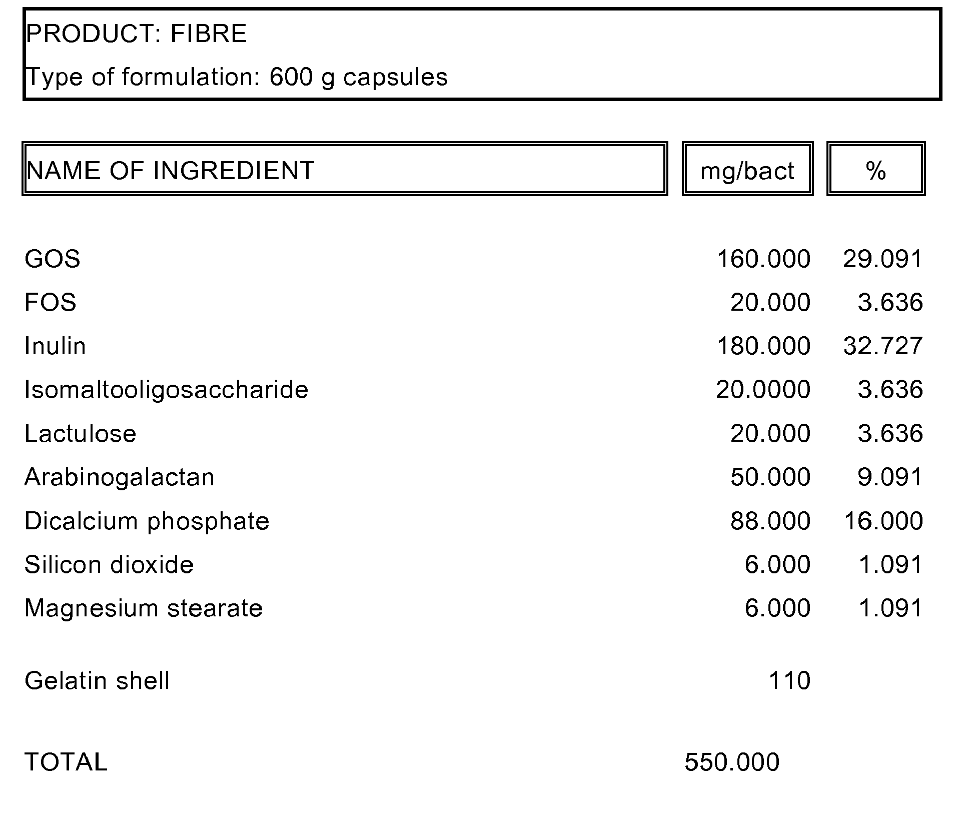

### Example 11

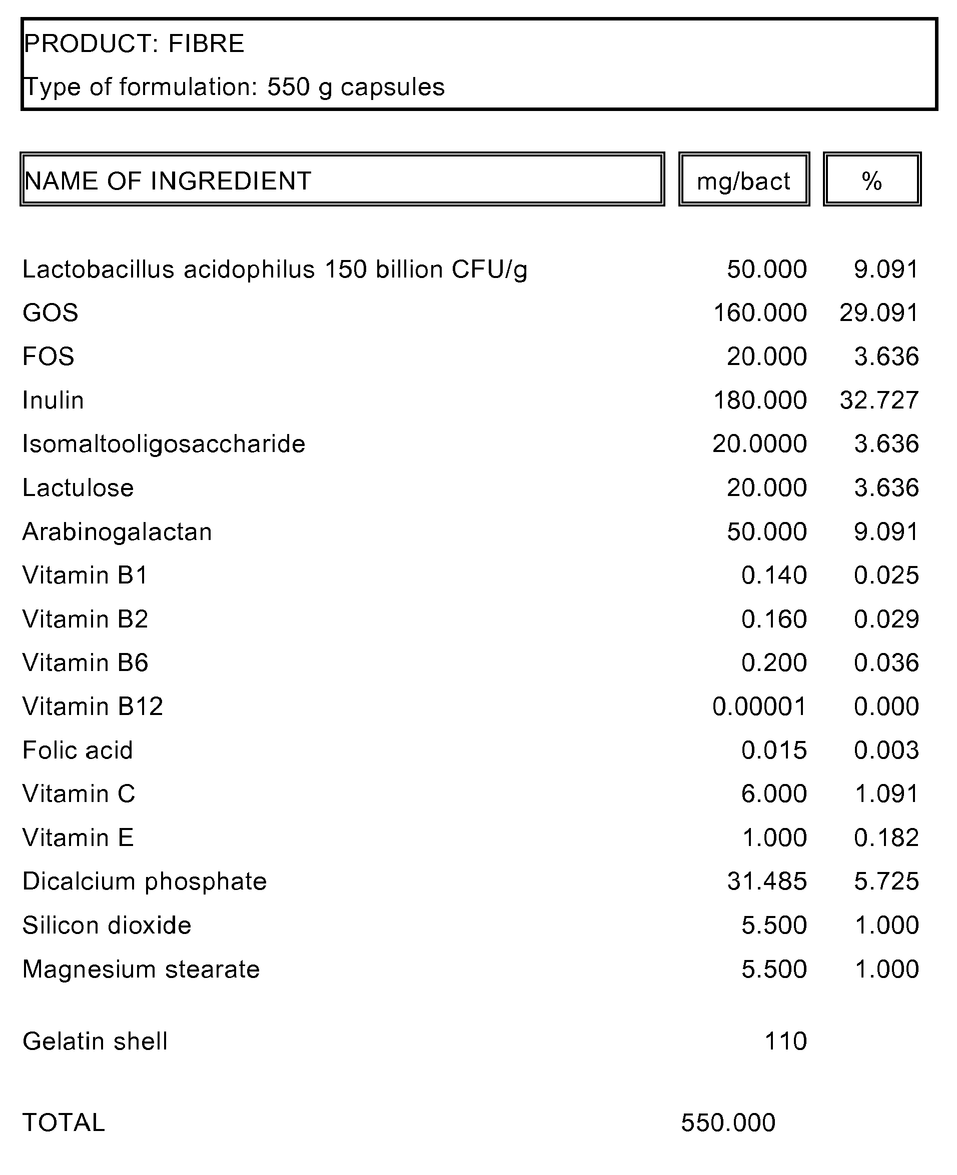

## Claims

1. Compositions containing:
a) one or more bifidogenic prebiotic ingredients chosen from inulin, fructooligosaccharides (FOS), galactooligosaccharides (GOS), lactosucrose, pyrodextrin, soy oligosaccharides and transgalactooligosaccharides,
b) one or more lactogenic prebiotic ingredients chosen from isomaltooligosaccharide, lactilol, lactulose, xylooligosaccharide, mannan oligosaccharide and polydextrose, and
c) one or more immunogenic ingredients chosen from arabinogalactan and derivatives thereof, plant extracts or extracts of fungi which contain them.

2. Compositions as claimed in claim 1, wherein the bifidogenic and lactogenic prebiotic ingredients are present in the proportion of 10:1, and contain FOS and GOS in the ratio of 1:9 and GOS and FOS combined in the ratio of 1:1 with inulin.

3. Compositions as claimed in claim 1 or 2, wherein the bifidogenic and lactogenic prebiotic ingredients are present in the following percentages, taking their global value as 100:
40% GOS
5% FOS
45% inulin
5% isomaltooligosaccharide
2.5% lactulose
2.5% polydextrose.

4. Compositions as claimed in claims 1-3, wherein the immunogenic ingredients are chosen from arabinogalactan and derivatives thereof, and are present in pure form or as plant extracts or extracts of fungi that contain them.

5. Compositions as claimed in claims 1-4, wherein the immunogenic ingredients are present in the total composition in the quantity of between 1 and 1000 mg.

6. Compositions as claimed in claims 1-4, wherein the various ingredients are present in the following percentage intervals:
a) bifidogenic prebiotic ingredients: between 10 and 10,000 mg,
b) lactogenic prebiotic ingredients: between 10 and 10,000 mg, and
c) immunogenic ingredients: between 10 and 10,000 mg.

7. Use of:
a) one or more bifidogenic prebiotic ingredients chosen from inulin, FOS, GOS, lactosucrose, pyrodextrin, soy oligosaccharides and transgalactooligosaccharides,
b) one or more lactogenic prebiotic ingredients chosen from isomaltooligosaccharide, lactilol, lactulose, xylooligosaccharide, mannan oligosaccharide and polydextrose, and
c) one or more immunogenic ingredients chosen from arabinogalactan and derivatives thereof, plant extracts or extracts of fungi which contain them.
to prepare compositions for the prevention and treatment of gastroenteric disorders.

8. Use as claimed in claim 7, wherein the gastroenteric disorders are due to dysbiosis and/or alterations of the normal intestinal flora of any etiology.
